# EUROPEAN PATENT APPLICATION

(11) **EP 2 293 060 A2**
(43) Date of publication of application: **09.03.2011**
(21) Application number: 10174742.6
(22) Date of filing: 31.08.2010
(51) Int. Cl.: G01N 33/00

(54) **Gas sensor**

(30) Priority: 01.09.2009 JP 2009201301; 13.07.2010 JP 2010158694
(71) Applicant: HORIBA, Ltd., Minami-ku Kyoto-shi Kyoto 601-8510 (JP)
(72) Inventor: Yamagishi, Yutaka, Kyoto Kyoto 601-8510 (JP); Shibuya, Kyoji, Kyoto Kyoto 601-8510 (JP)
(74) Representative: Horn, Friedemann Lorenz

(57) **Abstract**

The present invention is intended to provide a gas sensor that can detect low concentration NO₂ at a few ppb level with high sensitivity, and adapted to at least include: a silver catalyst member 4 that is provided inside thereof with a catalyst flow path through which a sample gas can pass, wherein at least on a surface of the catalyst flow path, silver is exposed; a first semiconductor gas sensor element 5 that is provided with a gas sensitive film including n-type oxide semiconductor; and a main flow path 2 that communicatively connects the silver catalyst member and the first semiconductor gas sensor element, to let the sample gas flow from the silver catalyst member toward the first semiconductor gas sensor element.

## Description

### Technical Field

The present invention relates to a gas sensor that can detect NO₂ with high sensitivity.

### Background Art

Ozone and NO₂ are substances that coexist in air with having concentrations of up to a few 100s ppb, and both controlled under Air Pollution Control Act of Japan. For this reason, the concentrations of the substances are required to be measured with high accuracy.

Recently, the present inventors have developed a semiconductor gas sensor element provided with a gas sensitive film including tungsten oxide (WO₃) (Patent literatures 1 and 2). In particular, it has been thought that the semiconductor gas sensor element formed with the gas sensitive film including monoclinic tungsten oxide (WO₃) containing hexagonal tungsten oxide (WO₃) crystal described in Patent literature 2 exhibits a high sensitivity for NO₂, and is therefore preferably usable also for measuring a concentration of NO₂ in air, which exists at a few ppb level. However, it has turned out that this semiconductor gas sensor element has a sensitivity for ozone of the order of a few 10s ppb coexisting with NO₂ in air, which reaches 100 times as high as that for NO₂.

On the other hand, conventionally, to measure concentrations of ozone and NO₂ in air, a chemiluminescence detection method (CLD method) and an ultraviolet absorption method (UV method) are respectively employed as official methods for measuring the NO₂ and ozone concentrations, and any of the methods enables an analysis target gas component of which a concentration in air is at a few 10s ppb level to be measured at a level of the order of a few ppb.

In the chemiluminescence detection method, with use of a phenomenon in which near-infrared light is emitted when NO₂* in an excited state that is generated by reacting NO with ozone from an ozone generator separately provided in an analyzer returns to NO₂ in a ground state, an amount of the light is measured to thereby measure the concentration of NO₂. As described, NO₂ cannot be directly detected, and therefore to measure the concentration of it, NO₂ is required to be once reduced to NO with an NO₂ converter using a catalyst, which is then required to be introduced to a reaction cell and reacted with high concentration ozone.

Also, in the ultraviolet absorption method, with use of the fact that an ozone absorption band exists in an ultraviolet region near 220 to 280 nm, the ozone concentration is measured.

For these reasons, to measure the concentrations of the both substances of ozone and NO₂ in air, it is necessary to use two types of analyzers, i.e., an NOx analyzer using the chemiluminescence detection method and an ozone analyzer using the ultraviolet absorption method, to separately measure the both substances; however, there is not known a single device that can simply measure the concentrations of ozone, NO₂, and further NO at a concentration level of the order of ppb in air.

Note that ozone has the absorption band in the ultraviolet region near 220 to 280 nm, whereas NO has a relatively narrow absorption band in an ultraviolet region near 230 nm, and NO₂ has a wide absorption band from an ultraviolet region near 300 nm to a visible region, and it is not easy to discriminate an absorption wavelength, particularly, between NO and ozone because an optical system becomes very complicated. For this reason, it is difficult to simply detect NO₂, ozone, and further NO concurrently with a single analyzer using the ultraviolet absorption method.

### Citation List

### Patent Literature

Patent literature 1: JP2007-64908A
Patent literature 2: International Publication No. 2009/034843

Therefore, the present invention is intended to provide a gas sensor that can detect low concentration NO₂ at a few ppb levels with high sensitivity.

### Summary of Invention

The present inventors have found that when, in order to remove ozone coexisting with NO₂ in air, we try a silver catalyst as an ozone scrubber, not only ozone can be well removed from air, but by making a sample gas brought into contact with the silver catalyst serve as a test target gas, detection sensitivity of a semiconductor gas sensor element for NO₂ can also be improved, and therefore reached the completion of the present invention.

That is, a gas sensor according to the present invention at least includes: a silver catalyst member that is provided with a catalyst flow path through which a sample gas can pass, wherein at least on a surface of the catalyst flow path, silver is exposed; a first semiconductor gas sensor element that is provided with a gas sensitive film including an n-type oxide semiconductor; and a main flow path that communicatively connects the silver catalyst member and the first semiconductor gas sensor element, to let the sample gas flow from the silver catalyst member toward the first semiconductor gas sensor element.

According to such a configuration, on an upstream side of the semiconductor gas sensor element, the silver catalyst member is provided, and the sample gas having passed through the silver catalyst member serves as a test target gas for the semiconductor gas sensor element, so that NO₂ in the sample gas can be detected with high sensitivity.

In the semiconductor gas sensor element formed with the gas sensitive film including an n-type oxide semiconductor, when oxidized gases such as ozone and NO₂ come into contact with the gas sensitive film, the oxidized gases are absorbed to the gas sensitive film to take surface electrons, so that a space charge layer of the gas sensitive film is increased, and consequently an electrical resistance of the gas sensitive film is increased. For this reason, depending on a concentration of a gas component to be analyzed, a resistance value of the gas sensitive film is varied, and therefore by sensing the variation in resistance value, the concentration of the gas component can be measured.

A mechanism by which, in the case of making the sample gas having passed through the silver catalyst member serve as the test target gas, a detection sensitivity of the semiconductor gas sensor element for NO₂ in the sample gas is significantly (approximately 10 times) increased is not clarified at the time of the present invention; however, as the mechanism, (1) NO₂ is activated by the silver catalyst (e.g., NO₂ is further oxidized to N₂O₅ to thereby improve electron trapping capability from the gas sensitive film), (2) the gas sensitive film is activated by silver particles released from the catalyst, or the like is presumed.

Also, in the case of targeting the sample gas containing ozone in air or the like for analysis, by passing the sample gas through the silver catalyst member, ozone in the sample gas is decomposed into oxygen, and thereby ozone can be removed from the sample gas, so that only NO₂ can be well detected with eliminating influence of ozone on the semiconductor gas sensor element.

As the gas sensitive film, one including n-type oxide semiconductor can be cited, such as tungsten oxide (WO₃), or tin oxide (SnO₂); however, among them, one including tungsten oxide (WO₃) is preferably used, and in particular, the semiconductor gas sensor element formed with the gas sensitive film including monoclinic tungsten oxide (WO₃) containing hexagonal tungsten oxide (WO₃) crystal expresses a high detection sensitivity for low concentration NO₂ at a few ppb level.

The gas sensor according to the present invention may further include a second semiconductor gas sensor element that is provided on an upstream side of the silver catalyst member through the main flow path and provided with a gas sensitive film including n-type oxide semiconductor. According to such a configuration, ozone can be detected with the second semiconductor gas sensor element, and NO₂ can be detected with the first semiconductor gas sensor element provided on a downstream side of the silver catalyst member, so that ozone and NO₂ can be simultaneously detected with the single gas sensor.

Further, the gas sensor according to the present invention may include: an oxidation catalyst member that is provided on a downstream side of the first semiconductor gas sensor element through the main flow path and oxidizes NO to NO₂; and a third semiconductor gas sensor element that is provided on a downstream side of the oxidation catalyst member through the main flow path and provieded with a gas sensitive film including n-type oxide semiconductor. According to such a configuration, NO in the sample gas is oxidized to NO₂ by the oxidation catalyst member, and therefore a total gas concentration of NO and NO₂ originally contained in the sample gas is detected as an NO₂ concentration. For this reason, from a difference between a resistance value of the third semiconductor gas sensor element and a resistance value of the first semiconductor gas sensor element, an NO concentration can be measured. Note that as the oxidation catalyst member, a member including, for example, platinum (Pt), manganese oxide (γ-MnO₂), or the like is cited.

On the other hand, the gas sensor according to the present invention may not include the second semiconductor gas sensor element, but instead may include a second flow path that branches from the main flow path; is provided in parallel with the silver catalyst member; and makes the sample gas reach the first semiconductor gas sensor element without passing the sample gas through the silver catalyst member. According to such a configuration, by alternately switching between flow of the sample gas into the silver catalyst member and flow of the sample gas into the second flow path, gas concentrations of both of ozone and NO₂ can be measured only with the single semiconductor gas sensor element.

Further, the gas sensor according to the present invention may include: an oxidation catalyst member that is provided between the silver catalyst member and the first semiconductor gas sensor element through the main flow path so as to communicatively communicate with them and oxidizes NO to NO₂; and a third flow path that is on a downstream side of the silver catalyst member; branches from the main flow path and that is provided in parallel with the oxidation catalyst member, to let the sample gas reach the first semiconductor gas sensor element without passing the sample gas through the oxidation catalyst member. According to such a configuration, by alternately switching between flow of the sample gas into the oxidation catalyst member and flow of the sample gas into the third flow path, three types of gas concentrations of ozone, NO₂, and NO can be measured only with the single semiconductor gas sensor element.

According to the present invention having such a configuration, low concentration NO₂ at a few ppb level can be detected with high sensitivity.

### Brief Description of Drawings

Fig. 1 is a schematic configuration diagram of a gas sensor according to a first embodiment of the present invention;
Fig. 2 is a plan view of an ozone detecting sensor element in the same embodiment;
Fig. 3 is a vertical cross-sectional view of the ozone detecting sensor element along an X-X line in Fig. 2;
Fig. 4 is a graph illustrating an NO₂ detection sensitivity improvement effect of a silver catalyst member;
Fig. 5 is a schematic configuration diagram of an experimental system for examining the sample gas flow rate dependence of the gas sensor sensitivity;
Fig. 6 is a graph illustrating a sample gas flow rate dependence of a gas sensor sensitivity;
Fig. 7 is a schematic configuration diagram of a gas analyzer using the gas sensor according to the same embodiment;
Fig. 8 is a schematic configuration diagram of a gas sensor according to a second embodiment of the present invention;
Fig. 9 is a schematic configuration diagram of a gas sensor according to a third embodiment of the present invention;
Fig. 10 is a schematic configuration diagram of a gas sensor according to a fourth embodiment of the present invention;
Fig. 11 is a schematic configuration diagram of a gas sensor according to another embodiment: and
Fig. 12 is a schematic configuration diagram of a gas sensor according to still another embodiment.

### Description of Embodiments

### <First embodiment>

A first embodiment of the present invention is described below with reference to the drawings.

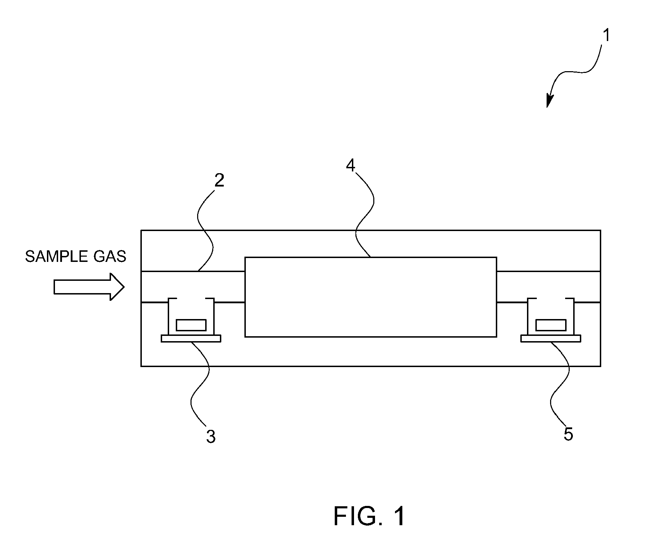

A gas sensor 1 according to the present embodiment is, as illustrated in Fig. 1, provided with: a main flow path 2 that is provided with an inlet and an outlet and lets a sample gas flow or circulate; and an ozone detecting sensor element 3 (corresponding to a second semiconductor gas sensor element), a silver catalyst member 4, and an NO₂ detecting sensor element 5 (corresponding to a first semiconductor gas sensor element) that are sequentially provided from an upstream side of the main flow path 2.

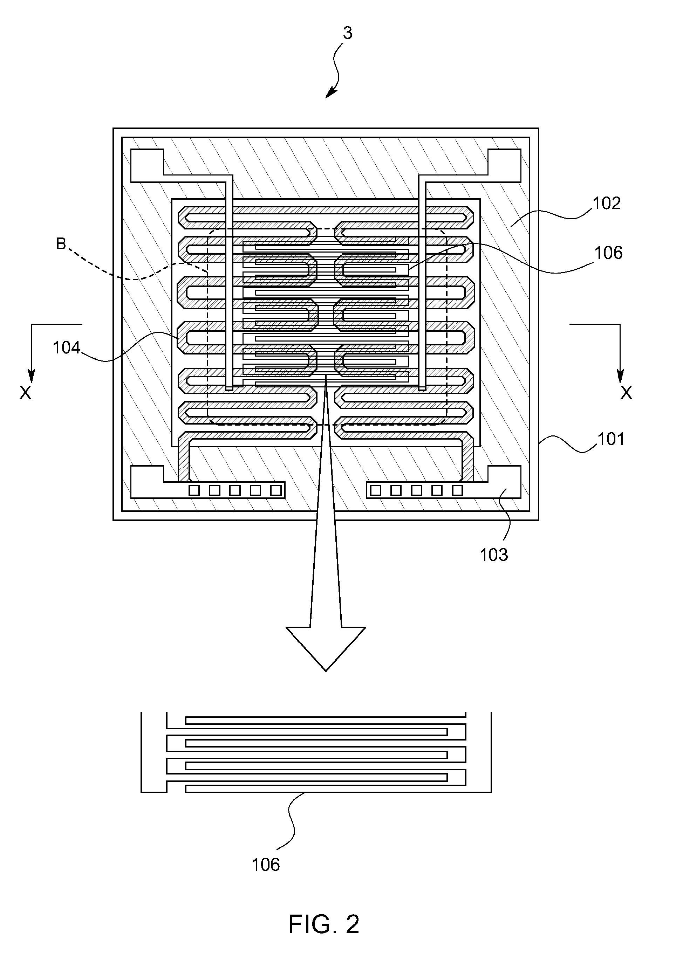

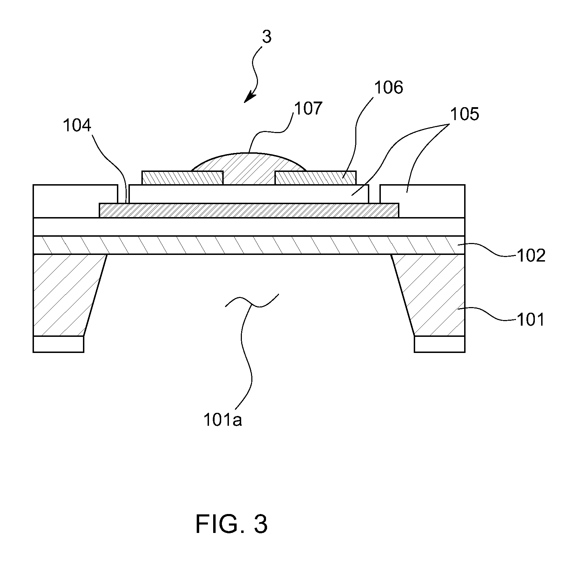

In the following, each of the parts is described in detail. The ozone detecting sensor element 3 is, as illustrated in Figs. 2 and 3, provided with: a silicon (Si) substrate 101 having in its central part a hollow part 101a that is rectangular shaped in a plan view; an SiO₂ insulating film 102 that is formed on the Si substrate 101 so as to block the hollow part 101a and has a rectangular shaped diaphragm structure; an energizing electrode 103 formed on the insulating film 102; a heater 104 applied with a constant voltage by the energizing electrode 103; an insulating film 105 that is formed by depositing non-silicate glass (NSG) on the heater 104 and then etching necessary portions; a resistance measuring electrode 106 formed on the insulating film 105; and a gas sensitive film 107 formed on the resistance measuring electrode 106.

The heater 104 is formed in an area corresponding to a substantially entire area of the rectangular shaped hollow part 101a in the Si substrate 101 on the insulating film 102 by etching a metal film substantially made of a hard-to-oxidize refractory material such as platinum (Pt) in a double zigzag pattern shape of which a density is maximum in its peripheral part and gradually decreases toward its central part. Specifically, the heater 104 is formed in the double zigzag pattern shape in which a heater line width and heater line pitch are both minimum in both sides where pieces of the rectangular shaped insulating film 102 face to each other, and the heater line width and pitch both gradually increase toward the central part, on the basis of which the heater 104 is configured such that when the heater 104 is energized and heated through the energizing electrode 103, a temperature of a whole of a rectangular area B surrounded by a dashed line on the insulating film 102 can be elevated to an uniform temperature on the basis of Joule heat. In addition, a material for the heater 104 is not limited to platinum if the material is a hard-to-oxidize refractory material, and as the material, tantalum (Ta) or tungsten (W) may be used.

The resistance measuring electrode 106 is substantially made of gold (Au) or the like, and as a main part extracted and clearly illustrated in the lower part of Fig. 2, formed in a comb-like pattern occupying a substantially entire area within the uniform temperature area B based on the heater 104.

The gas sensitive film 107 includes monoclinic tungsten oxide (WO₃) containing hexagonal tungsten oxide (WO₃) crystal, and is formed so as to cover a most part on the resistance measuring electrode 106 having the comb-like pattern. Such a gas sensitive film 107 is formed by sintering a monoclinic WO₃ suspension containing hexagonal WO₃ crystal on the resistance measuring electrode 106. Alternatively, the gas sensitive film 107 may be formed by a method such as reactive sputtering or reactive vacuum evaporation.

The ozone detecting sensor element 3 as described above has a fine MEMS structure of which one side has a length of 1 to 2 mm.

As the NO₂ detecting sensor element 5, the same semiconductor gas sensor element as the ozone detecting sensor element 3 is also used.

In the gas sensor 1 according to the present embodiment, the semiconductor gas sensor element formed with the gas sensitive film 107 including monoclinic WO₃ containing hexagonal WO₃ crystal, which is used for the ozone detecting sensor element 3 and the NO₂ detecting sensor element 5, is one that can detect ozone or NO₂ on the basis of a variation in resistance value thereof due to a contact between the gas sensitive film 107 and ozone or NO₂; however, a sensitivity (resistance value) for ozone is approximately 100 times as high as a sensitivity for NO₂.

The silver catalyst member 4 includes silver wool, and on the basis of catalytic action of silver, reduces ozone (O₃) in the sample gas having passed through an inside thereof to oxygen (O₂). The silver catalyst member 4 is provided with an unillustrated heater, and heated to, for example, 80 °C in a temperature range of 50 to 150 °C by the heater.

When air in which ozone and NO₂ coexist is flowed as the sample gas through the main flow path 2 of the gas sensor 1 according to the present embodiment, the ozone detecting sensor element 3 first detects ozone.

Specifically, the ozone detecting sensor element 3 detects both of ozone and NO₂; however, the sensitivity of the ozone detecting sensor element 3 for ozone is approximately 100 times the sensitivity for NO₂, and when an ozone concentration is high, an NO₂ concentration tends to be low, so that in the case of using typical air as the sample gas, an influence of NO₂ on a resistance value indicated by the ozone detecting sensor element 3 can be regarded as being approximately 1 % or less. For this reason, the ozone detecting sensor element 3 may be assumed to substantially detect ozone.

In addition, when the NO₂ concentration is high and the ozone concentration is low, the ozone concentration can be measured by subtracting a resistance value indicated by the NO₂ detecting sensor element 5 from a resistance value indicated by the ozone detecting sensor element 3. Note that, in the case of the resistance value subtraction, it is necessary to take into account an increase in NO₂ sensitivity due to the silver catalyst member 4.

Subsequently, when the sample gas passes through the silver catalyst member 4, ozone in the sample gas is reduced to oxygen, on the basis of which ozone is removed from the sample gas. Then, when the sample gas from which ozone is removed comes into contact with the gas sensitive film 107 of the NO₂ detecting sensor element 5, NO₂ is detected.

The present inventors have clarified that, at this time, by making the sample gas having passed through the silver catalyst member 4 serve as a test target gas for the NO₂ detecting sensor element 5, a detection sensitivity of the NO₂ detecting sensor element 5 for NO₂ in the sample gas is significantly improved.

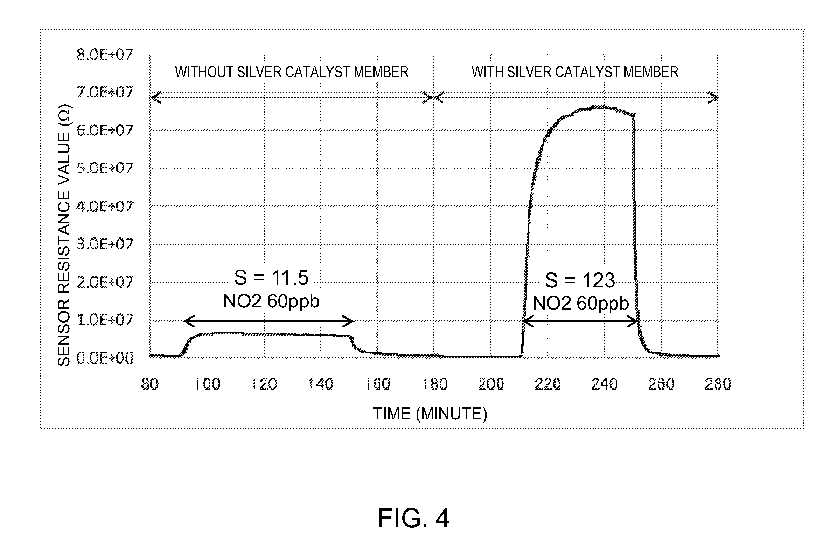

In order to examine such an improvement effect of the detection sensitivity for NO₂ by the silver catalyst member 4, with use of NO₂ gas (NO₂ concentration: 60 ppb) as the sample gas, the gas sensor 1 provided with the silver catalyst member 4 including the silver wool and a gas sensor not provided with the silver catalyst member 4 are used to measure resistance values indicated by the gas sensitive films 107 of the respective NO₂ detecting sensor elements 5, and a result of the measurements is illustrated in Fig. 4. As illustrated in Fig. 4, the result shows that the case of providing the silver catalyst member 4 including the silver wool exhibits a resistance value (sensitivity) as high as an approximately 10 times. Incidentally, when NO₂ concentrations in the sample gases exhausted from the respective gas sensors were measured with an NOx analyzer incorporating an NOx converter using the chemiluminescence detection method, the NO₂ concentrations were both 60 ppb. Note that, in Fig. 4, "S" represents a sensitivity of the semiconductor gas sensor element, and defined by S = Rg / Ra (Ra: a resistance value in air, and Rg: a resistance value in the test target gas at some concentration).

Thus, according to the gas sensor 1 configured as above according to the present embodiment, even if ozone and NO₂ coexist in the sample gas, concentrations of these two substances can be measured with the single gas sensor 1.

Also, in the present embodiment, by making the sample gas having passed through the silver catalyst member 4 serve as the test target gas for the NO₂ detecting sensor element 5, the detection sensitivity of the NO₂ detection sensor element 5 for NO₂ in the sample gas can be significantly improved. Note that a mechanism by which the detection sensitivity of the NO₂ detecting sensor element 5 for NO₂ in the sample gas having passed through the silver catalyst member 4 is improved is not clarified at the time of the present invention; however, as the mechanism, (1) NO₂ is activated by the silver catalyst (e.g., NO₂ is further oxidized to N₂O₅ to thereby improve electron trapping capability from the gas sensitive film 107), (2) the gas sensitive film 107 is activated by silver particles released from the catalyst, or the like is presumed.

Further, the sensor element 3 or 5 has the fine structure of which one side has the length of 1 to 2 mm, and therefore even if the silver catalyst member 4 is provided, the small sized gas sensor 1 can be built.

Also, the present inventors have found that sensitivities of the ozone detecting sensor element 3 and NO₂ detecting sensor element 5 are influenced by a flow rate, and have clarified that by increasing the flow rate of the sample gas supplied to the sensor elements 3 and 5, the sensitivities are increased.

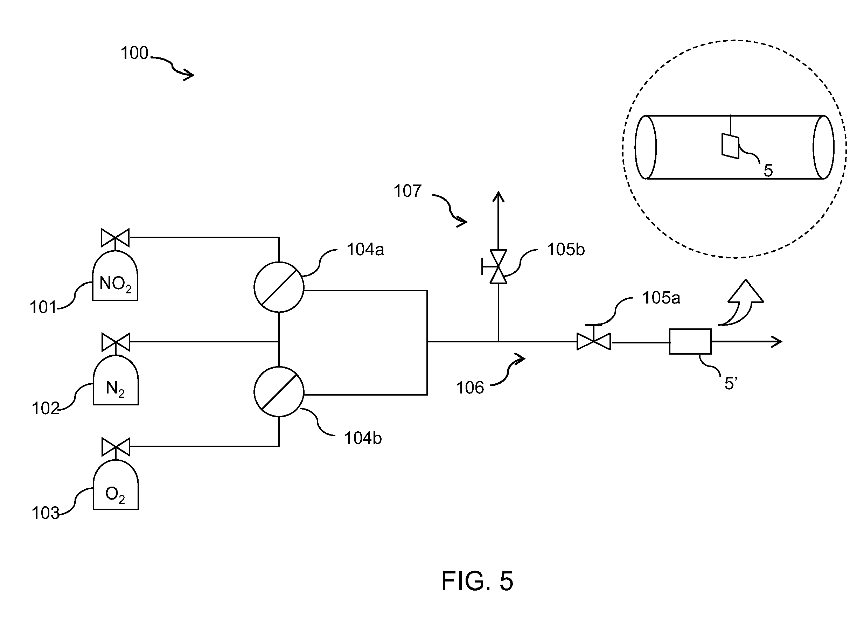

Subsequently, in order to examine the flow rate dependence of the sensitivity of the NO₂ detecting sensor element 5, an experimental system 100 as illustrated in Fig. 5 is fabricated. The experimental system 100 is provided with, as NO₂, N₂, and O₂ supply sources 101, 102, and 103, high pressure cylinders in which the respective gases are filled, and the respective gases supplied from the gas supply sources are mixed in dividers 104a and 104b to achieve predetermined concentrations, and thereby regulated to a sample gas. The obtained sample gas (250 mL/min) passes through a main flow path 106, and is then supplied into a cell 5' provided with the NO₂ detecting sensor element 5. The NO₂ detecting sensor element 5 is incorporated in the cell 5' having a cylindrical shape (inner diameter φ 7 mm) with being hung so as to be easily influenced by a flow rate of the sample gas. On an upstream side of the cell 5', a needle valve 105a is provided, and the flow rate of the sample gas is regulated by the needle valve 105a. Also, an exhaust path 107 that branches from the main flow path 106 and is intended to exhaust the sample gas is provided, and also provided with a needle valve 105b.

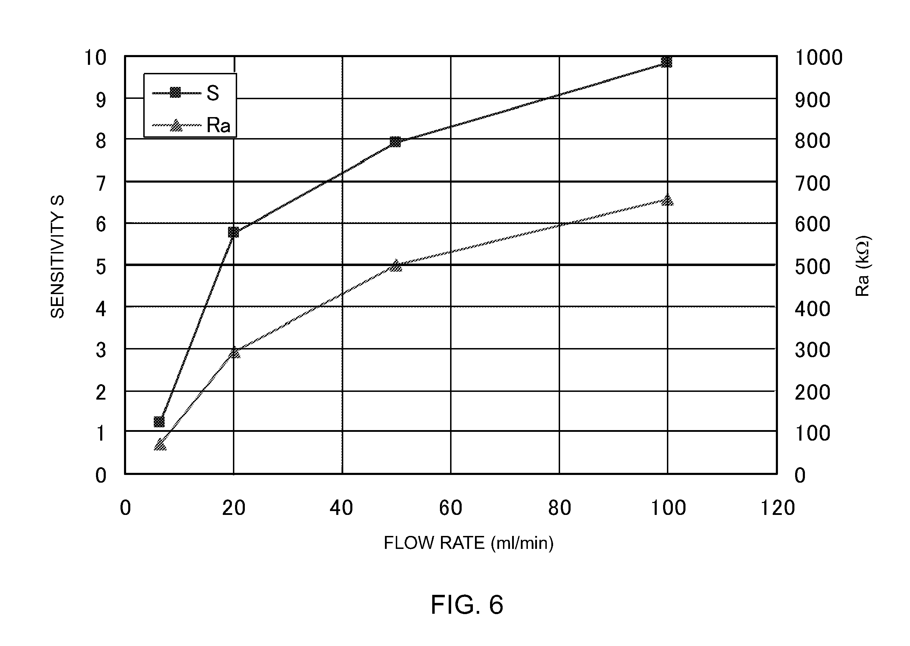

Such an experimental system 100 is used to supply the sample gas (N₂ concentration: 80 vol%, O₂ concentration: 20 vol%) in which the NO₂ concentration is regulated to 50 ppb to the NO₂ detecting sensor element 5 while adjusting the needle valve 105a to vary the flow rate of the sample gas. While varying the flow rate of the sample gas from 100, to 50, to 6.5, to 20 mL/min, sensor response is continuously measured to obtain sensitivities (resistance values) of the NO₂ detecting sensor element 5 at the respective flow rates. Note that, at this time, when the flow rate of the sample gas is varied, a temperature of the Pt heater provided in the NO₂ detecting sensor element 5 is also varied, and therefore, in order to observe only the influence of the flow rate, the experiment is carried out with adjusting a heater voltage to keep constant (200 °C) the temperature of the Pt heater. An obtained result is illustrated in a graph of Fig. 6.

It turns out from the graph illustrated in Fig. 6 that as the flow rate of the sample gas is decreased, the sensitivity (S) and the resistance value (Ra) in the zero gas of the NO₂ detecting sensor element 5 decrease, and in a smaller flow rate range, a degree of the decrease in resistance value becomes larger. Accordingly, it can be expected from the result that if the flow rate of the sample gas is increased to some extent, the sensitivity is saturated. By operating the gas sensor 1 according to the present invention in a flow rate region where the sensitivity is saturated, more stable and higher sensitivity sensor response can be expected. In addition, in the present experimental system, if the flow rate is equal to or more than 10 mL/min, a sensitivity necessary for the measurement can be obtained. Also, in Fig. 6, "S" representing the sensitivity is defined in the same manner as that in Fig. 4.

Further, a gas analyzer 10 configured with use of the gas sensor 1 according to the present embodiment is described below with reference to the drawings.

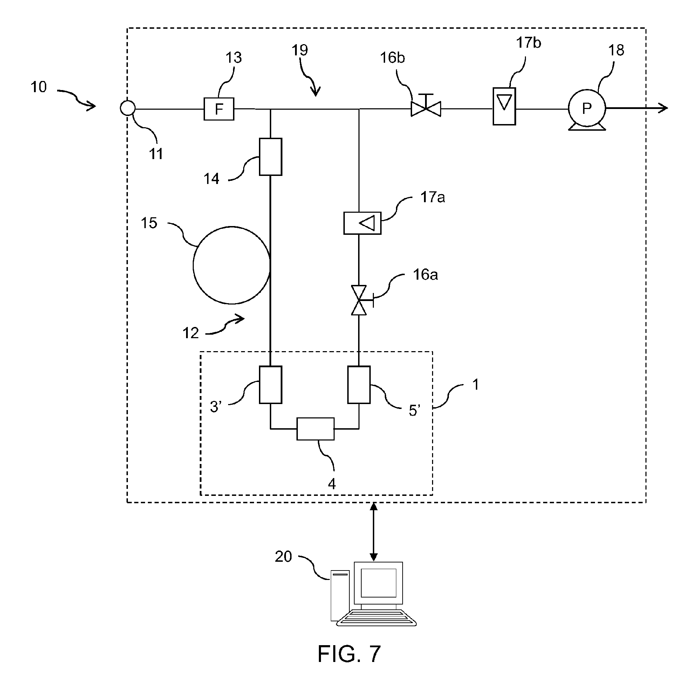

The present gas analyzer 10 is, as illustrated in Fig. 7, provided with: a flow path system that lets a sample gas flow or circulate; the gas sensor 1 that is provided in the flow path system and measures concentrations of ozone and NO₂; and an information processor 20 that collects measurement result data from the gas sensor 1 and controls flow rate regulators and the like arranged in the flow path system.

The flow path system includes a main flow path 12 through which the sample gas flows, and the main flow path 12 is opened at an upstream end thereof as an inlet port 11 and allocated with a suction pump 18 on a most downstream side thereof.

In the main flow path 12, subsequent to the inlet port 11, from the upstream side, a filter 13 that removes dust and the like, a temperature/humidity sensor 14, a heating tube 15, the ozone detecting sensor element 3, the silver catalyst member 4, the NO₂ detecting sensor element 5, a first flow rate regulator 16a, a first flowmeter 17a, a second flow rate regulator 16b, a second flowmeter 17b, and the suction pump 18 are arranged in series in this order. Among them, the ozone detecting sensor element 3 and the NO₂ detecting sensor element 5 are respectively provided in cells 3' and 5', and an assembly of them and the silver catalyst member 4 corresponds to the gas sensor 1 according to the present embodiment.

The temperature/humidity sensor 14 is one that is intended to measure a relative humidity in the introduced sample gas at an ambient temperature; further obtain an absolute humidity from a value of the relative humidity; and on the basis of a value of the absolute humidity, correct measurement result data obtained by the gas sensor 1.

The heating tube 15 is one that is intended to heat the sample gas, for example, from a room temperature to approximately 60 °C, and has a length of, for example, approximately 1 m. By letting the sample gas flow or circulate in such a long tube to gradually heat it, the sample gas can be heated to a uniform temperature. However, if, at the time when the sample gas is introduced from the inlet port 11, the sample gas is already heated to the predetermined temperature, the heating tube 15 is unnecessary.

The heating tube 15, the ozone detecting sensor element 3, the silver catalyst member 4, and the NO₂ detecting sensor element 5 are all adjusted in temperature by an after-mentioned control part, and controlled to make measurements at a constant temperature.

The first and second flow rate regulators 16a and 16b are ones intended to regulate a flow rate of the sample gas flowing through the main flow path 12, as which specifically needle valves are respectively used. Note that the flow rate regulator 16 is not limited to the needle valve if the flow rate can be regulated, and as the flow rate regulator 16, for example, a capillary, mass flow controller, or the like may be used.

Also, the present gas analyzer 10 is provided with the first and second flowmeters 17a and 17b, and can thereby check the flow rate; however, for example, by providing a flowmeter outside the present gas analyzer 10, the first and second flowmeters 17a and 17b can also be omitted.

A point between the filter 13 and the temperature/humidity sensor 14, and a point between the first flowmeter 17a and the second flow rate regulator 16b are short-circuited by a branched path 19. Also, at the point between the first flowmeter 17a and the second flow rate regulator 16b, the sample gas flows through the branched path 19 joins the sample gas flowing through the main flow path 12; however, in the present embodiment, the flow rate of the sample gas flowing through the main flow path 12 is 50 to 100 mL/min, whereas the flow rate of the sample gas flowing through the branched path 19 is 500 to 1000 mL/min. For this reason, due to viscosity of the sample gas, the sample gas flowing through the main flow path 12 is pulled by the sample gas flowing through the branched path 19, on the basis of which the flow rate of the sample gas flowing through the main flow path 12 can be increased. Accordingly, by providing such a branched path 19, high speed response becomes possible.

The main flow path 12 and the branched path 19 are respectively formed of fluororesin tubes that are superior in corrosion resistance, and also filter 13 is applied with fluororesin coating.

The information processor 20 is a general-purpose or dedicated device provided with, in addition to a CPU, input means such as a memory and a keyboard, output means such as a display, and the like. Also, by, according to a predetermined program stored in the memory, collaboratively operating the CPU and its peripheral devices, the information processor 20 fulfills functions as the control part that performs open/close control of the valves provided in the flow path system and temperature control of the heaters; a calculation processing part that applies predetermined calculation processing to pieces of measurement result data obtained from the temperature/humidity sensor 14 and gas sensor 1 to calculate concentrations of ozone and NO₂ in the sample gas; and the like.

### <Second embodiment>

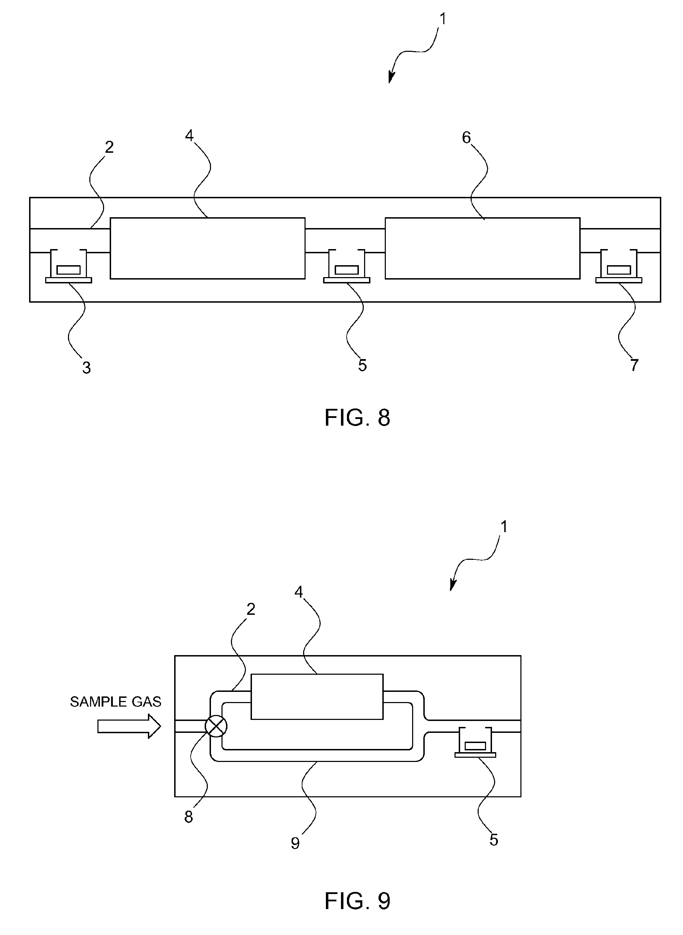

A second embodiment of the present invention is described below with reference to Fig. 8. Note that, in the following, the description is provided with focusing on differences from the first embodiment.

A gas sensor 1 according to the second embodiment is, as illustrated in Fig. 8, further provided with, on a downstream side of an NO₂ detecting sensor element 5, an oxidation catalyst member 6 and an NO detecting sensor element 7 (corresponding to a third semiconductor gas sensor element) that are sequentially provided from an upstream side.

The oxidation catalyst member 6 includes platinum (Pt) wool, manganese oxide (γ-MnO₂) wool, or the like, and is one intended to oxidize NO in a sample gas to NO₂.

As the NO detecting sensor element 7, the same semiconductor gas sensor element as an ozone detecting sensor element 3 and an NO₂ detecting sensor element 5 is used.

In the gas sensor 1 according to the present embodiment, NO in the sample gas having passed through the oxidation catalyst member 6 is oxidized to NO₂, and therefore in the NO detecting sensor element 7, both of NO and NO₂ originally contained in the sample gas are detected as NO₂. For this reason, by subtracting a resistance value indicated by the NO₂ detecting sensor element 5 from a resistance value indicated by the NO detecting sensor element 7, a concentration of NO can be measured.

Thus, according to the gas sensor 1 configured as above according to the present embodiment, even if ozone, NO₂, and NO coexist in the sample gas, concentrations of these three substances can be measured with the single gas sensor 1.

### <Third embodiment>

A third embodiment of the present invention is described below with reference to Fig. 9. Note that, in the following, the description is provided with focusing on differences from the first embodiment.

A gas sensor 1 according to the third embodiment is configured such that, as illustrated in Fig. 9, the ozone detecting sensor element 3 is not provided, but in parallel with a silver catalyst member 4, a second flow path 9 that makes a sample gas directly reach an NO₂ detecting sensor element 5 is provided, and at a branching point between a main flow path 2 and the second flow path 9, a first switching valve 8 is provided to be able to alternately switch between flow of the sample gas into the silver catalyst member 4 and flow of the sample gas into the second flow path 9 at intervals of, for example, a few minutes.

In the gas sensor 1 according to the present embodiment, by switching the first switching valve 8 such that the sample gas passes through the silver catalyst member 4, ozone in the sample gas having passed through the silver catalyst member 4 is reduced to oxygen, on the basis of which ozone is removed from the sample gas, and therefore NO₂ can be detected with the NO₂ detecting sensor element 5. On the other hand, by switching the first switching valve 8 such that the sample gas passes through the second flow path 9, ozone can be detected with the NO₂ detecting sensor element 5.

Thus, according to the gas sensor 1 configured as above according to the present embodiment, by switching the first switching valve 8, even if ozone and NO₂ coexist in the sample gas, concentrations of these two substances can be measured only with the single NO₂ detecting sensor element 5.

### <Fourth embodiment>

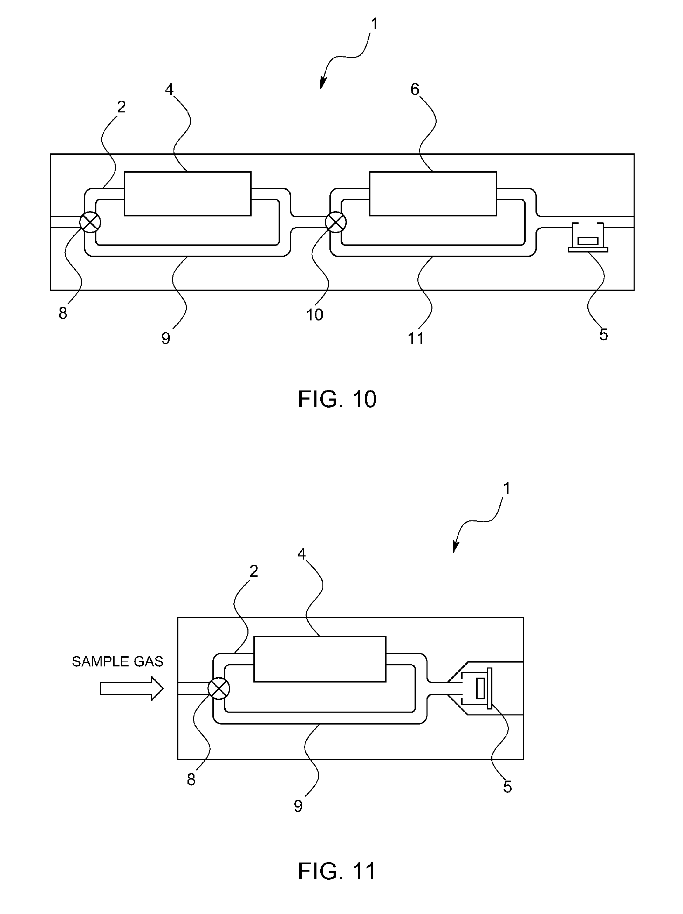

A fourth embodiment of the present invention is described below with reference to Fig. 10. Note that, in the following, the description is provided with focusing on differences from the third embodiment.

In a gas sensor 1 according to the fourth embodiment, as illustrated in Fig. 10, between the silver catalyst member 4 and the NO₂ detecting sensor element 5 in the gas sensor 1 according to the third embodiment, and on a downstream side of a joining point between a main flow path 2 and a second flow path 9, an oxidation catalyst member 6 is further provided, and in parallel with the oxidation catalyst member 6, there is provided a third flow path 11 that makes a sample gas directly reach an NO₂ detecting sensor element 5 without passing the sample gas through the oxidation catalyst member 6. Also, the gas sensor 1 is configured such that, at a branching point between the main flow path 2 passing through the oxidation catalyst member 6 and the third flow path 11, a second switching valve 10 is provided to be able to alternately switch between flow of the sample gas into the oxidation catalyst member 6 and flow of the sample gas into the third flow path 11 at intervals of, for example, a few minutes.

In the gas sensor 1 according to the present embodiment, by switching the second switching valve 10 such that the sample gas passes through the oxidation catalyst member 6, NO in the sample gas having passed through the oxidation catalyst member 6 is oxidized to NO₂, and therefore in the NO₂ detecting sensor element 5, both of NO and NO₂ originally contained in the sample gas are detected as NO₂. On the other hand, by switching the second switching valve 10 such that the sample gas flows through the third flow path 11, NO₂ or ozone can be detected in the NO₂ detecting sensor element 5. Then, by subtracting a resistance value of the NO₂ detecting sensor element 5 for the case where only NO₂ is detected from a resistance value of the NO₂ detecting sensor element 5 for the case where both of NO and NO₂ are detected as NO₂, a concentration of NO can be measured.

Thus, according to the gas sensor 1 configured as above according to the present embodiment, by switching the switching valves 8 and 10, even if ozone, NO₂, and NO coexist in the sample gas, concentrations of these three substances can be measured only with the single NO₂ detecting sensor element 5.

Note that the present invention is not limited to any of the above-described embodiments.

For example, in the above-described embodiments, the respective substances contained in the sample gas are brought into contact with the gas sensitive film 107 by diffusion; however, as illustrated in Fig. 11, it may be configured such that the sample gas is directly blown to the gas sensitive film 107.

The silver catalyst member 4 is provided inside with catalyst flow paths, for example gaps, through which the sample gas can pass, and not limited to the silver wool but if at least on surfaces of the catalyst flow paths, silver is exposed, any substance may be employed, for example, a porous carrier carrying and supporting silver particles, or the like may be employed..

The gas sensor 1 according to the present invention is one in which, by making the sample gas having passed through the silver catalyst member 4 serve as a test target gas for the NO₂ detecting sensor element 5, a detection sensitivity of the NO₂ detecting sensor element 5 for NO₂ in the sample gas is significantly improved, and therefore the gas sensitive film 107 of the NO₂ detecting sensor element 5 is not limited to one including monoclinic WO₃ containing hexagonal WO₃ crystal, but there may be employed a gas sensitive film 107 including only monoclinic WO₃, which is inferior in detection sensitivity for low concentration NO₂ as compared with the gas sensitive film 107 including monoclinic WO₃ containing hexagonal WO₃ crystal.

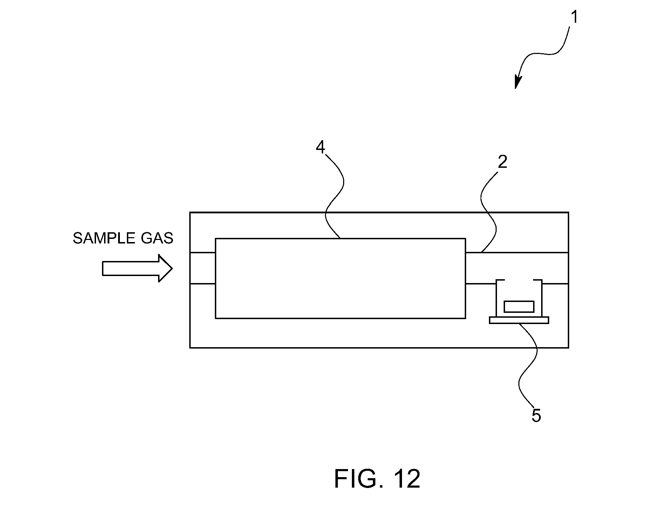

Also, in the case where it is only necessary to be able to measure a concentration of NO₂ in the sample gas, but it is not necessary to measure a concentration of ozone, the gas sensor 1 may have a configuration in which, as illustrated in Fig. 12, from the gas sensor 1 according to the first embodiment, the ozone detecting sensor element 3 is removed.

Besides, it should be appreciated that parts or all of the above-described embodiments and variations may be appropriately combined, and various modifications can be made without departing from the scope of the present invention.

### Industrial Applicability

Thus, according to the present invention, low concentration NO₂ can be detected with high sensitivity.

### Reference Signs List

1: Gas sensor
2: Main flow path
4: Silver catalyst member
5: NO₂ detecting sensor element (first semiconductor gas sensor element)

## Claims

1. A gas sensor at least comprising:
a silver catalyst member that is provided with a catalyst flow path through which a sample gas can pass, wherein at least on a surface of the catalyst flow path, silver is exposed;
a first semiconductor gas sensor element that is provided with a gas sensitive film including an n-type oxide semiconductor; and
a main flow path that communicatively connects the silver catalyst member and the first semiconductor gas sensor element, to let the sample gas flow from the silver catalyst member toward the first semiconductor gas sensor element.

2. The gas sensor according to claim 1, wherein the gas sensitive film includes tungsten oxide.

3. The gas sensor according to claim 1 or 2, further comprising a second semiconductor gas sensor element that is provided on an upstream side of the silver catalyst member through the main flow path and provided with a gas sensitive film including n-type oxide semiconductor.

4. The gas sensor according to claim 1 or 2, further comprising a second flow path that branches from the main flow path and that is provided in parallel with the silver catalyst member, to let the sample gas reach the first semiconductor gas sensor element without passing the sample gas through the silver catalyst member.
